# EUROPÄISCHE PATENTANMELDUNG

(11) **EP 2 077 119 A1**
(43) Veröffentlichungstag der Anmeldung: **08.07.2009**
(21) Anmeldenummer: 07450239.4
(22) Anmeldetag: 21.12.2007
(51) Int. Cl.: A61K 38/55, A61K 38/48

(54) **Behandlung von Fibrosen und Lebererkrankungen**

(71) Anmelder: Apeiron Biologics Forschungs- und Entwicklungsgesellschaft M.B.H., 1230 Wien (AT)
(72) Erfinder: Loibner, Hans, 1230 Wien (AT); Schuster, Manfred, 2191 Schrick (AT); Österreicher, Christoph, 1090 Wien (AT)
(74) Vertreter: Sonn & Partner Patentanwälte

(57) **Zusammenfassung**

Die vorliegende Erfindung betrifft ACE2 zur therapeutischen Behandlung oder Vorbeugung einer Fibrose oder Lebererkrankung.

## Beschreibung

Die vorliegende Erfindung betrifft das Gebiet der Behandlung von Fibrosen und Lebererkrankungen.

Fibrosen sind Krankheiten, die durch die Bildung fibrotischen Gewebes oder Gewebeschäden gekennzeichnet sind. Dabei kommt es zu einer krankhaften Vermehrung von Bindegewebszellen im Bindegewebe selbst oder auch in einem Organ. Dabei wird das Gewebe des betroffenen Organes verhärtet, es entstehen narbige Veränderungen, die im fortgeschrittenen Stadium zur Einschränkung der jeweiligen Organfunktion führen.

Unter Fibrose versteht man daher die überschießende Vermehrung des Bindegewebes aller menschlichen Organe, deren Ursache in der Überproduktion von Proteinen der extrazellulären Matrix, hauptsächlich von Kollagen liegt. Die komplexen molekularen Regulationsabläufe, die zu dieser Überproduktion führen, sind nur ansatzweise bekannt. Bis heute konnten jedoch zahlreiche Auslöser identifiziert werden, wie z.B. toxische Substanzen, Wachstumsfaktoren, Peptidfragmente von Matrixproteinen, Hormone u.a., die Fibroblasten, wie Myofibroblasten und Stellatzellen, als Zielzellen der erhöhten Bildung von Matrixproteinen stimulieren.

Die Leber ist äußerst stoffwechselaktiv und ein hochregeneratives Organ, das auch bei hohen Schädigungen noch in der Lage ist neue Leberzellen zu bilden und sich dabei zu regenerieren. Bei Leberkrankungen kommt es dabei - je nach Intensität - zu eine starken Gewebeneubildung, wobei auch ein starkes Risiko der Bildung von fibrotischem Gewebe besteht.

Es ist ein Ziel der vorliegende Erfindung Entwicklungen, die zu Fibrosen und Lebererkrankungen führen, vorzubeugen, deren Voranschreiten zu bremsen und Fibrosen und Lebererkrankungen zu behandeln.

Die Erfindung betrifft daher ein Protein oder eine Nukleinsäure, welche das Protein kodiert, wobei das Protein ACE2 ist, zur therapeutischen Behandlung oder Vorbeugung einer Lebererkrankung oder Fibrose. Ebenso betrifft die vorliegende Erfindung die Verwendung von einem ACE2 Protein oder einer ACE2 kodierenden Nukleinsäure zur Herstellung einer pharmazeutischen Zusammensetzung zur Behandlung oder Vorbeugung von Lebererkrankung oder Fibrose.

Angiotensin Converting Enzyme 2 (ACE2) ist ein essentielles Enzym des Renin-Angiotensin-Aldosteron Systems, welches als membranverankertes Glykoprotein auf diversen Organen, wie Herz, Niere, Leber und Lunge, aber auch auf Blutgefäßen exprimiert wird. ACE2 wurde 1997 als ACE homologes Enzym entdeckt (Genbank Acc.: BAB40370, kodiert durch eine Nukleinsäure mit der Sequenz nach Genbank Acc.: AB046569). Anfänglich wurde ihm dieselbe enzymatische Aktivität wie ACE zugerechnet (US 6,989,363). Erst später wurde entdeckt, dass ACE2 eine gänzlich andere, sogar antagonistische Wirkungsweise zu ACE aufweist (WO 2004/000367). ACE2 ist eine Carboxypeptidase, die zahlreiche Peptidsubstrate mit stark unterschiedlicher Selektivität und Aktivität spaltet. ACE2 ist auch ein zellulärer Bindungspartner von SARS-Coronaviren. Die Herunterregulierung von ACE2 oder die Verabreichung von ACE2, um Virus-Rezeptoren zu blockieren, kann daher die Suszeptibilität ACE2 präsentierender Zellen mindern (WO 2006/122819). Die beschriebenen Funktionen für ACE2 sind vor allem die Umwandlung von Ang II zu Ang 1-7, wobei Substrat und Produkt dieser Reaktion antagonistische Eigenschaften aufweisen. Ang II wirkt im Wesentlichen vasokonstriktiv und blutdrucksteigernd. Ang 1-7 wirkt vasodilatorisch und weist bei Nieren-, Lungen- und Herzerkrankungen einen Schutzeffekt auf (WO 2004/000367). Das ACE2 Produkt Ang 1-7 hemmt im Weiteren ACE, das Enzym, welches für die Produktion von Ang II maßgeblich verantwortlich ist. Das Renin Angiotensinsystem spielt eine wesentliche Rolle in der Pathologie von Lebererkrankungen, im Speziellen der Leberfibrose. Die Anwesenheit von Ang II ist für pro-fibrogene Effekte in Stellatzellen der Leber (HSCs, hepatitic stellate cells) verantwortlich. Es konnte gezeigt werden, dass die Expression und die Aktivität von ACE2 in unter chronischer HCV Infektion leidenden Patienten zunimmt. Hierbei handelt es sich anscheinend um einen Schutzmechanismus, der allerdings nicht ausreicht, um eine Regeneration des Organs einzuleiten. Eine Steigerung der ACE2 Aktivität ist daher zielführend.

Erfindungsgemäß kann durch die Inhibierung der Fibrosebildung einer Fibrose vorgebeugt werden, bzw. durch die Verhinderung des Verschlechterns der Fibrose der Heilungsprozess beschleunigt werden. Eine prophylaktische Therapie ist daher mit ACE2 oder einer ACE kodierenden Nukleinsäure möglich. Eine solche prophylaktische Therapie soll jedoch nicht in einem absoluten Sinn verstanden werden, sondern lediglich so, dass das Risiko des Auftretens einer Fibrose gesenkt wird oder die Fibrosesymtome bzw. die Intensität einer sich entwickelnden Fibrose gemildert werden. Insbesondere betrifft die vorliegende Erfindung die Behandlung oder Vorbeugung des Fortschreitens einer Fibrose oder Lebererkrankung. Eine prophylaktische Anwendung ist insbesondere bei Risikopatienten, mit einer erhöhten Wahrscheinlichkeit eine Fibrose oder Lebererkrankung zu entwickeln (im Vergleich zu gesunden Individuen), wie z.B. Alkoholikern oder Patienten mit einer Hepatitis C Infektion, sinnvoll.

In bevorzugten Ausführungsformen ist die Fibrose eine lokale Fibrose eines Gewebes oder Organs. Solche Organ spezifischen Fibrosen sind Leberfibrosen, Lungenfibrosen, Bindegewebsfibrosen, insbesondere Fibrose der Muskelsepten, Nierenfibrose und die Fibrose der Haut, z.B. in Kombination einer Entzündung - Sklerodermie. Vorzugsweise ist die Fibrose eine Fibrose eines inneren Organs, z.B. der Leber, Niere, Lunge, des Herzens, des Magens, des Darms, des Pankreas, einer Drüse, eines Muskels, eines Knorpels von Sehnen und Bändern oder eines Gelenks. Eine spezielle Form der Fibrose ist die zystische Fibrose oder eine rheumatische Fibrose.

Bevorzugt ist die Fibrose auf eine exzessive Ablagerung der Komponenten der extrazellulären Matrix, insbesondere Proteine, wie Kollagen, zurückzuführen. Kollagen ist ein Strukturprotein des Bindegewebes, insbesondere der extrazellulären Matrix. Die Kollagenbildung, insbesondere in Verbindung mit dem Marker SMA (smooth muscle actin) korreliert direkt mit dem Voranschreiten der Fibrose. Erfindungsgemäß konnte eine besonders effektive Inhibierung der Kollagen-Ablagerung durch ACE2 beobachtet werden.

Des Weiteren ist, basierend auf dem allgemeinen Mechanismus, auch die Behandlung von chronischen Fibrosen möglich. Insbesondere kann die Fibrose durch mechanische oder chemische Zell- oder Gewebeschäden oder Wunden, Krebs oder Tumore, Infektionen, insbesondere von Pathogenen, wie Viren, Bakterien oder Pilzen, durch Implantate, inklusive Organimplantate, sowie Medikamente hervorgerufen sein. Infektionen können z.B. Organ-spezifisch sein, wie z.B. Hepatitis Virus Infektion, insbesondere durch HCV. Weitere vorzugsweise fibrotische Erkrankungen, die erfindungsgemäß mit ACE2 oder einer ACE2 kodierenden Nukleinsäure behandelt werden können, sind z.B. primäre oder sekundäre Fibrosen, insbesondere Fibrosen, die durch eine Autoimmunreaktion hervorgerufen werden, Morbus Ormond, retroperitoneale Fibrose.

Die Leber ist ein äußerst stoffwechselaktives und hochregeneratives Organ, das auch bei hohen Schädigungen noch in der Lage ist neue Leberzellen zu bilden und sich dabei zu regenerieren. Bei Lebererkrankungen kommt es dabei - je nach Intensität - zu einer starken Gewebeneubildung, wobei auch ein starkes Risiko der Bildung von fibrotischem Gewebe besteht. Somit eignet sich die vorliegende Anwendung von ACE2 oder einer ACE2 kodierenden Nukleinsäure zur Behandlung von Lebererkrankungen, insbesondere um fibrotischen Symptomen als Nebeneffekt oder Hauptindikation vorzubeugen oder zu behandeln. Zudem konnte gezeigt werden, dass ALT (Alanin Aminotransaminase), ein Indikator für die Leberfunktion durch ACE2 Behandlung, signifikant erhöht werden konnte. Daher eignet sich die vorliegende Erfindung insbesondere zur Herstellung oder Bewahrung der Leberfunktion bei einer Lebererkrankung. In speziellen Ausführungformen steht die Lebererkrankung mit Leberschäden oder Leberzellschäden in Verbindung.

In speziellen Ausführungsformen tritt die Fibrose oder Lebererkrankung zusammen mit einer Inflammation auf, z.B. bei einer Hepatitis (entzündliche Lebererkrankung). Inflammationen oder Entzündungen diverser Organe oder Gewebe heilen oft zumindest teilweise schlecht ab und können auch zur Bildung von fibrotischem Gewebe führen. Eine inflammatorische Reaktion ist ein Prozess, bei dem sich Abwehrzellen auf den Weg zu einer Infektionsquelle machen und dort die Beseitigung der Ursache sicherstellen. Diese Inflammation kann also z.B. durch eine Infektion verursacht sein. Unterschiedliche Mittlerstoffe werden hierbei freigesetzt, die zu der Beseitigung beitragen, aber auch die Entzündungssymtome erzeugen. Bei Fehlregulierungen der Reaktion können diese Symptome den hauptsächlichen Schaden verursachen bzw. generell die Krankheitsquelle sein. Inflammationen können auch künstlich verursacht werden, z.B. bei Organtransplantationen, die letztendlich zur Abstoßung des Fremdorgans führen können. Ebenso können Inflammationen als Nebenwirkung durch bestimmte Medikamente hervorgerufen werden.

Die Expression von ACE2 wird durch diverse Stimuli gesteuert. Es wurde nun auch gefunden, dass ACE2 durch das Auftreten inflammatorischer Zytokine, wie TNF-alpha, IFN-gamma oder IL-4, herunterreguliert wird, was in weiterer Folge zu diversen Erkrankungen und zu einer Akkumulation von Ang II in den betroffenen Kompartimenten und zu einer Potenzierung der eingeleiteten Immunantwort führt. Zytokine dienen im Wesentlichen der Kommunikation diverser Zelltypen des Immunsystems. Üblicherweise besteht einer der ersten Schritte einer naszierenden Inflammation darin, dass antigene Substanzen durch Antigen-präsentierende Zellen (APCs) aufgenommen und als fremd eingestuft werden. In weiterer Folge kommt es zu einem ersten Ausstoß inflammatorischer Zytokine durch die betroffenen APCs, welche dadurch weitere Zellen des Immunsystems alarmieren. Dieser Mechanismus ist hoch reguliert und kontrolliert, um eine Immunantwort nur dann einzuleiten, wenn diese auch tatsächlich berechtigt ist, und diese wieder abzudrehen, wenn die antigene Substanz neutralisiert wurde. Dennoch kann es vorkommen, dass diese eingeleitete Immunantwort außer Kontrolle gerät und sich gegen den eigenen Organismus wendet. Die Akkumulation von Ang II, z.B in diversen Nieren-, Herz- und Lungenerkrankungen, bedingt eine fortschreitende Inflammation und auch eine gesteigerte Infiltration des betroffenen Gewebes durch Zellen des Immunsystems und in weiterer Folge ein Überschießen der Immunantwort. Eine Schlüsselstelle ist hierbei allerdings immer die zelluläre Immunantwort als Reaktion auf einen Stimulus, welche in einer sich potenzierenden Amplifikationskaskade den primären Zweck, eine fremde Substanz zu neutralisieren, bei weitem übererfüllt und in weiterer Folge den Organismus schädigt.

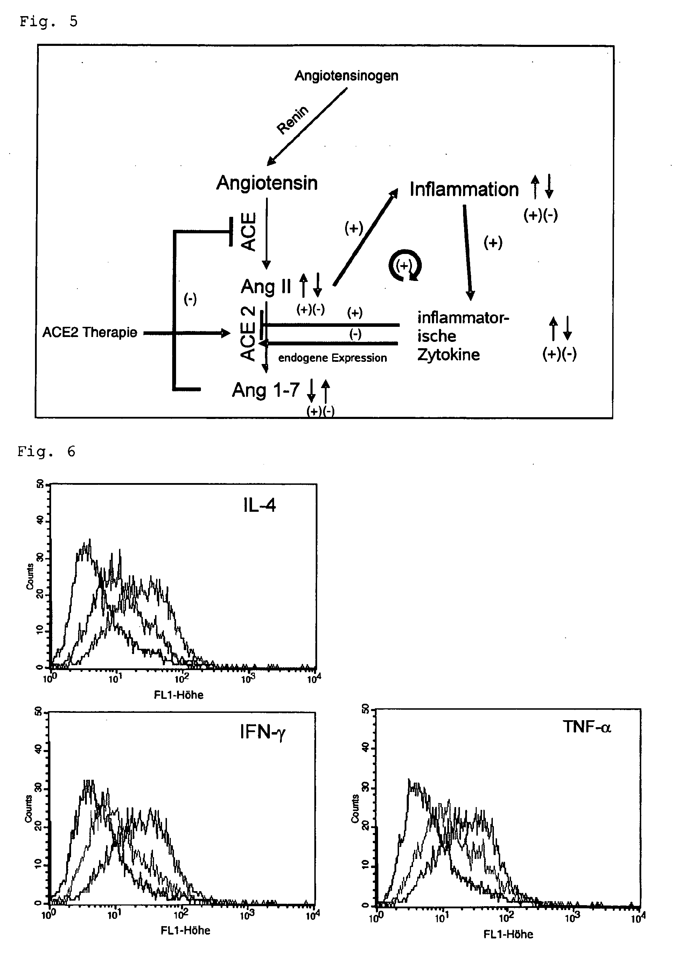

Der erste Schritt der aufkeimenden Immunantwort ist die Aussendung inflammatorischer Signale in Form von Zytokinen. Wesentliche Vertreter hierfür sind zum Beispiel IL-4, IFN-gamma oder TNF-alpha. Substanzen, die die Eigenschaft haben, diese Zytokinexpression nach Stimulierung der Immunzelle zu unterbinden oder abzuschwächen, sind brauchbare Therapeutika zur Attenuierung einer überschießenden Immunantwort. Die ACE2 Expression nimmt in Gegenwart inflammatorischer Zytokine auf zellulärerer Ebene stark ab, was zu einer Potenzierung der Inflammation vor allem durch Akkumulation von Ang II, durch die Abnahme von Ang 1-7 und durch das dadurch bedingte Ausbleiben einer Herabsetzung der Ang II Nachbildung führt. Die deswegen stark zunehmenden Ang II Konzentrationen fördern aufgrund der stark inflammatorischen Eigenschaften von Ang II die weitere Potenzierung der Inflammation, welche in weiterer Folge zu einer noch deutlicheren Attenuierung der ACE2 Expression führt. Um diesem Teufelskreis zu entkommen, wird erfindungsgemäß ACE2 therapeutisch verabreicht und somit einer Ang II Akkumulation vorgebeugt und dabei die Inflammation gedämpft: ACE2 verringert unmittelbar die hohen Ang II Titer, was die durch Ang II sich laufend steigernde Inflammation abschwächt. Ang 1-7 wird nachgebildet und schwächt durch dessen anti-inflammatorische Wirkung ebenfalls die Inflammation ab. Weiters limitiert Ang 1-7 durch dessen Eigenschaft, ACE zu inhibieren, die Nachproduktion von Ang II. Das Abflauen der Inflammation bewirkt, dass die ausgeschütteten Zytokine auf ein Normalmaß zurückkehren, und dass es wieder zu einer endogenen ACE2 Expression kommt, die nachhaltig für den Abbau von Ang II und die Entstehung von Ang 1-7 sorgt und wieder zu einem stabilen funktionellen RAS führt. In weiterer Folge stellt sich wieder ein sich selbst regulierendes beständiges Gleichgewicht der agierenden Komponenten des RAS ein. Eine erneute ACE2 Gabe kann somit gänzlich ausbleiben, wenn der ursprüngliche Stimulus des Immunsystems neutralisiert wurde. Eine schematische Darstellung der erwähnten Mechanismen ist in Fig. 5 wiedergegeben. Durch Gabe von ACE2 wird ein Ausweg aus der sich potenzierenden Inflammation geschaffen.

Die in den Beispielen angeführten Daten lassen die folgenden Rückschlüsse auf die Wirkung von ACE2 als Immunregulator zu. Bedingt durch einen antigenen Stimulus kommt es zur Ausschüttung inflammatorischer Zytokine. In Gegenwart inflammatorischer Zytokine kommt es zu einem Verlust der ACE2 Expression. In Abwesenheit von ACE2 akkumuliert das pro-inflammatorische Peptid Ang II, da es nicht durch ACE2 abgebaut werden kann. In Abwesenheit von ACE2 akkumuliert ebenfalls das pro-inflammatorische Zytokin TNF-alpha. ACE2 besitzt anti-inflammatorische Eigenschaften und setzt in Lymphozyten die Expression inflammatorischer Zytokine herab. Eine therapeutische ACE2 Gabe kompensiert daher die verlorengegangene endogene ACE2 Expression und kann eine aufkeimende Inflammation durch Herabsetzung von Ang II Titern, durch Bildung von Ang 1-7 und durch andere Effekte abfangen. Eine therapeutische ACE2 Gabe ermöglicht es sogar im Falle einer schweren Sepsis unter kontinuierlicher LPS Infusion Ang II Titer wieder auf das Niveau eines Gesunden herabzusetzen und die Regulierung des RAS dementsprechend wieder herzustellen. Eine therapeutische ACE2 Gabe ermöglicht es ferner im Falle einer schweren Sepsis unter kontinuierlicher LPS Infusion TNF-alpha Titer wieder auf das Niveau des Gesunden herabzusetzen. Derselbe Effekt konnte ebenfalls im Falle einer mechanischen, massiven Lungenschädigung durch Mekoniumaspiration beobachtet werden.

Vorzugsweise ist das Protein rekombinantes ACE2. ACE2 Sequenzen sind hinreichend bekannt, und es kann problemlos durch Einbringung von geeigneten Vektoren, welche ACE2 kodieren, in Expressionssystemen, insbesondere eukaryotischen, produziert werden. Solche Systeme sind z.B. Säugerzelllinien, wie CHO (Chinese Hamster Ovary) Zellen und NS0 Mauszellen oder Insektenzellen, z.B. Sf9. Zur Expression kann ein solcher Vektor bestimmte Zell-spezifische oder allgemeine Promotoren aufweisen.

Vorzugsweise ist das Protein (für das auch die ACE2 Nukleinsäure kodiert) wasserlösliches ACE2, insbesondere ohne Membrandomäne. Die menschliche ACE2 Sequenz ist durch die SEQ ID NO:1 angegeben:

MSSSSWLLLSLVAVTAAQSTIEEQAKTFLDKFNHEAEDLFYQSSLASWNYNTNITEENVQNM-
NNAGDKWSAFLKEQSTLAQMYPLQEIQNLTVKLQLQALQQNGSSVLSEDKSKRLNTILNTMS-
TIYSTGKVCNPDNPQECLLLEPGLNEIMANSLDYNERLWAWESWRSEVGKQLRPLYEEYVVLKN
EMARANHYEDYGDYWRGDYEVNGVDGYDYSRGQLIEDVEHTFEEIKPLYEHLHAYVRAKLM-
NAYPSYISPIGCLPAHLLGDMWGRFWTNLYSLTVPFGQKPNIDVTDAMVDQAWDAQRIF-
KEAEKFFVSVGLPNMTQGFWENSMLTDPGNVQKAVCHPTAWDLGKGDFRILMCTKVTMDDFLTA
HHEMGHIQYDMAYAAQPFLLRNGANEGFHEAVGEIMSLSAATPKHLKSIGLLSPDFQEDNE-
TEINFLLKQALTIVGTLPFTYMLEKWRWMVFKGEIPKDQWMKKWWEMKREIVGVVEPVPHDE-
TYCDPASLFHVSNDYSFIRYYTRTLYQFQFQEALCQAAKHEGPLHKCDISNSTEAGQKLFNMLR
LGKSEPWTLALENVVGAKNMNVRPLLNYFEPLFTWLKDQNKNSFVGWSTDWSPYADQSIK-
VRISLKSALGDKAYEWNDNEMYLFRSSVAYAMRQYFLKVKNQMILFGEEDVRVANLK-
PRISFNFFVTAPKNVSDIIPRTEVEKAIRMSRSRINDAFRLNDNSLEFLGIQPTLGPPNQPPVS

Dabei wird die autologe Signalsequenz (unterstrichen) durch die Wirtszelle für die Ausschleusung abgespalten. Vorzugsweise umfasst daher das erfindungsgemäße ACE2 Protein eine ACE2 Sequenz entsprechend der SEQ ID NO: 1 ab der Position 18. In weiteren Ausführungsformen weist das ACE2 Polypeptid keine transmembrane Domäne auf. Diese Transmembrandomäne befindet sich am C-Terminus der SEQ ID NO:1. Es handelt sich daher dann um lösliches ACE2. Besonders bevorzugte Ausführungsformen umfassen dabei lösliche ACE2-Polypeptide, deren Polypeptidkette aus den Aminosäuren die SEQ ID NO:1 bis zur Aminosäureposition 740 umfassen, oder enzymatisch aktive Fragmenten davon. Ein weiteres lösliches ACE2 Protein besteht aus den Aminosäuren 18-615 der SEQ ID NO: 1.

Die Löslichkeit eines Proteins wird nicht nur durch seine Aminosäuresequenz, sondern auch durch seine Faltung sowie durch post-translationelle Modifikationen bestimmt. Es sind vor allem geladene Zuckerstrukturen, die maßgeblich die Löslichkeit eines Proteins steigern und dessen pharmakologisches Profil beeinflussen. Der lösliche Abschnitt von ACE2 enthält 7 N-Glykosylierungsstellen. Vorzugsweise sind mindestens 80% der möglichen N-Glykosylierungspositionen glykosyliert und/oder das ACE2 Protein weist einen Zuckeranteil von größer als 10% (Massen-% des gesamten ACE2) oder 11%, 12%, 13%, 14%, vorzugsweise größer als 15% oder 16%, 17%, 18%, 19%, insbesondere größer als 20 % oder 21%, 22%, 23% 24% oder 25%, auf.

Obwohl menschliches ACE2 für die meisten Ausführungsformen bevorzugt ist, wird auch ACE2 von Maus, Ratte, Hamster, Schwein, Primaten oder Rind ermöglicht. ACE2 ist ein universelles Enzym in allen Säugetieren mit dem identischen Substrat Ang II. Daher ist es auch in Fremdorganismen einsetzbar. Somit können mit dem erfindungsgmäßen Protein (oder seiner Nukleinsäure) unabhängig vom Ursprung von ACE2 z.B. Menschen, Mäuse, Ratten, Hamster, Schweine, Primaten oder Rinder behandelt werden.

Erfindungsgemäß kann eine pharmazeutische Zusammenfassung umfassend das ACE2 Protein oder eine ACE2 kodierende Nukleinsäure zur Verfügung gestellt werden. Solche Zusammensetzungen können pharmazeutisch geeignete Salze derselben, zusätzlich Puffer, Tonizitätskomponenten oder pharmazeutisch geeignete Träger umfassen. Insbesondere ACE2 Nukleinsäuren können in geeigneten therapeutischen Vektorsystemen vorgesehen werden. Pharmazeutische Träger-Substanzen dienen der besseren Verträglichkeit der Zusammensetzung und ermöglichen bessere Löslichkeit sowie bessere Bioverfügbarkeit der Wirksubstanzen. Beispiele hierfür sind Emulgatoren, Verdickungsmittel, Redoxkomponenten, Stärke, Alkohollösungen, Polyethylenglycol oder Lipide. Die Auswahl eines geeigneten pharmazeutischen Trägers hängt stark von der Art der Verabreichung ab. Für orale Verabreichungen können flüssige oder feste Träger verwendet werden, für Injektionen sind flüssige Endzusammensetzungen erforderlich.

Vorzugsweise umfasst das erfindungsgemäß zu verwendende Medikament Puffersubstanzen oder tonische Substanzen. Mittels Puffer kann der pH-Wert des Medikaments auf physiologische Konditionen eingestellt werden und weiters können pH-Schwankungen abgeschwächt bzw. gepuffert werden. Ein Beispiel hierfür ist ein Phosphatpuffer. Tonische Substanzen dienen zur Einstellung der Osmolarität und können ionische Substanzen, wie zum Beispiel anorganische Salze, wie NaCl, oder auch nicht-ionische Substanzen, wie zum Beispiel Glycerin oder Kohlenhydrate, umfassen.

Bevorzugterweise ist die erfindungsgemäß zu verwendende Zusammensetzung zur systemischen, topischen, oralen oder intranasalen Verabreichung geeignet hergerichtet. Diese Verabreichungsformen des Medikaments der vorliegenden Erfindung ermöglichen eine schnelle und unkomplizierte Aufnahme. Zur oralen Verabreichung können beispielsweise feste bzw. flüssige Medikamente direkt oder aufgelöst bzw. verdünnt eingenommen werden.

Das erfindungsgemäß zu verwendende Medikament ist vorzugsweise zur intravenösen, intraarteriellen, intramuskulären, intravaskulären, intraperitonealen oder subkutanen Verabreichung geeignet hergerichtet. Hierfür eignen sich beispielsweise Injektionen oder Transfusionen. Verabreichungen direkt in die Blutbahn haben den Vorteil, dass die Wirkstoffe des Medikaments im gesamten Körper verteilt werden und die Zielgewebe schnell erreichen.

Die vorliegende Erfindung wird durch die folgenden Figuren und Beispiele illustriert ohne darauf beschränkt zu sein.

### Figuren:

Fig. 1: Kollagenbildung in der Leber von Wild Type und ACE2 knock-out Mäusen nach 21 tägiger BDL gemessen durch Sirius Rot Färbung (links) und mRNA Bestimmung (rechts) im Vergleich zu einer Kontrollgruppe.
Fig. 2: Messung des SMA Gehalts in Lebergewebe (A) und dessen mRNA (B) in der Leber von wild-Typ Mäusen und ACE2 knockout Mäusen nach 21 tägiger BDL im Vergelich zu einer Kontrollgruppe.
Fig. 3: Messung des SMA Gehalts in Lebergewebe (A) und dessen mRNA (B) in der Leber von wild-Typ Mäusen und ACE2 knockout Mäusen nach 21 tägiger BDL im Vergleich zu einer Kontrollgruppe.
Fig. 4: BDL Modell in wild-Typ Mäusen: Messung des ALT Gehalts in Serumproben von unbehandelten wild-Typ Mäusen und solchen, die eine ACE2 Therapie erhielten.
Fig. 5: Schematische Darstellung der Wiederherstellung eines funktionellen RAS durch ACE2 Therapie. Rote (+) Pfeile stellen Effekte der ausufernden Immunreaktivität dar, während blaue (-) Pfeile Änderungen bedingt durch die ACE2 Therapie bezeichnen.
Fig. 6: ACE2 spezifische FACS Analytik von Vero E6 Zellpräparationen nach 48 stündiger Inkubation mit 10 ng/ml IL-4, IFN-gamma oder TNF-alpha (Kurven mit mittlerem Peak) im Vergleich zu einer unstimulierten Kotrollgruppe (rote Kurven mit Peak rechts) und einer Kontrollserie (schwarze Kurven mit Peak links).
Fig. 7: Messung von TNF-alpha in PBMC Kulturüberständen 16 Stunden nach Stimulation mit LPS, PHA und LPS + PHA, ohne (schwarze Balken, links) oder in Gegenwart von ACE2 (graue Balken, mitte) oder ACE2 und Ang II (blaue Balken, rechts).
Fig. 8: Gemessene Ang II Konzentrationen in einem LPS induzierten Sepsismodell in Schweinen: blaue Kurve: APN 01 (rACE2) behandelte Tiere, graue Kurve: Placebo behandelte Tiere, graue Kurve (schwarze Punkte): gesunde Tiere nach APN 01 Administration.
Fig. 9: Gemessene ACE2 Aktivität in Mäusen, Schweinen und Rhesus Makaken.
Fig. 10: TNF-alpha Serumkonzentration in einem LPS induzierten Sepsismodell in Schweinen. ACE2 behandelte Tiere wurden in Blau, Placebo behandelte Tiere in Grau dargestellt. TNF- alpha Konzentrationen wurden auf die jeweiligen Ausgangswerte zu Therapiebeginn (100%) normiert.
Fig. 11: TNF- alpha Serumkonzentration in einem durch mekoniumaspirationinduzierten ARDS Modell in Schweinen. ACE2 behandelte Tiere wurden in Blau, Placebo behandelte Tiere in Grau dargestellt.

### Beispiele:

### Beispiel 1: Leberfibrosemodelle, Bedeutung von ACE2 in der Leberfibrose

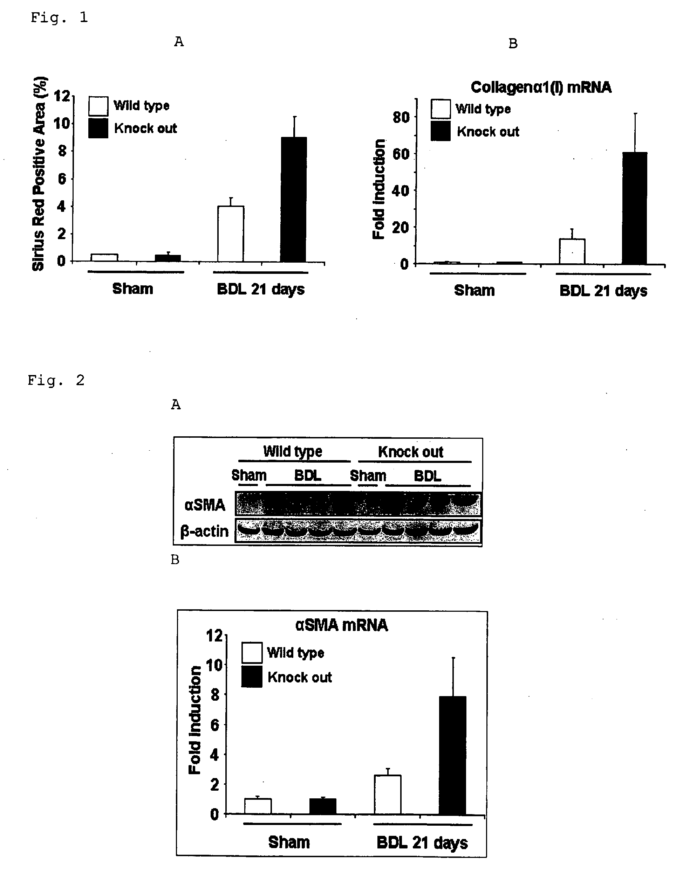

ACE2 knock-out und ACE2 wild type Mäuse wurden nach Abschnürung des Gallenganges (Bile Duct Ligation, BDL) nach 21 [?] ausgewertet und mit Sham-Kontrollgruppen verglichen. Die pathologische Untersuchung der Leber zeigte deutlich erhöhte Kollagenablagerungen in den Tieren, die der BDL unterzogen wurden (Fig. 1). Die Kollageneinlagerung im Lebergewebe wurde durch spezifische Färbung mit Sirius Rot untersucht und war erstaunlicherweise in ACE2 knockout Tieren 2,5 fach höher als in der Wildtypegruppe (Fig. 1). Die Anzahl an Kollagen produzierenden Zellen der Leber wurde durch Messung von SMA, einem Marker für aktivierte Stellatzellen, mittels Westernblot und mRNA Messung bestimmt. Fig. 2 stellt den Zusammenhang zwischen fehlender ACE2 Aktivität und Leberschädigung dar und zeigt deutlich, dass die Anzahl Kollagen produzierender Zellen deutlich erhöht ist.

Dieser Ansatz zeigt, dass es eine Korrelation zwischen der Abwesenheit von ACE2 und der Kollagenablagerung in der geschädigten Leber gibt. Die Kollagenablagerung ist ein wesentliches pathologisches Symptom der fortschreitenden Leberschädigung.

### Beispiel 2: Therapeutisches Modell

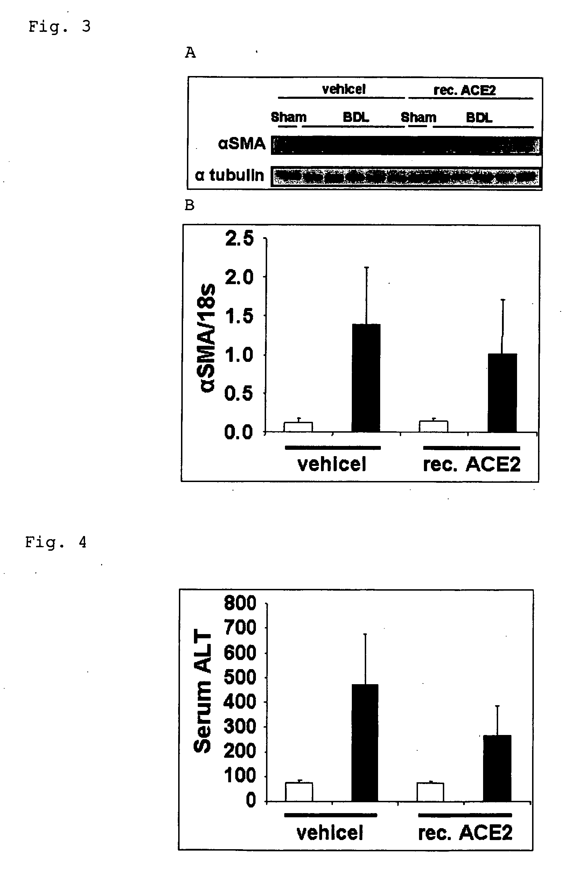

In einem zweiten Ansatz wurde wild-type Mäusen nach erfolgter BDL für 14 Tage lang rekombinantes ACE2 intravenös als tägliche Bolusinjektion von 2 mg/kg verabreicht. Diese Tiere wurden abermals mit einer Kontrollgruppe, die nur eine Salzlösung verabreicht bekam, nach Ende der Therapie verglichen: Fig. 3 zeigt sehr deutlich, dass die SMA Konzentration im Gewebe und somit die Anzahl an Kollagen produzierenden Zellen im geschädigten Lebergewebe der Wildtype Tiere sehr deutlich zunimmt, während SMA in der Leber von ACE2 behandelten Mäusen mittels Western Blot nicht nachweisbar war. Die Analytik der mRNA von SMA bestätigt dieses Ergebnis. In Fig. 4 ist die ALT Serumkonzentration der untersuchten Gruppen zum Ende des Experimentes dargestellt. Es könnte hier ebenfalls gezeigt werden, dass ALT in der ACE2 behandelten Gruppe niedrigere Konzentrationen erreichte.

Beide Studien zeigen sehr deutlich auf, dass eine reduzierte ACE2 Aktivität zu einer Verschlechterung der Lebersymptome führt. Höhere ACE2 Aktivität reduziert die Anzahl Kollagen produzierender Zellen sowie die Kollagenanreicherung im Gewebe. Es konnte ferner ein therapeutischer Effekt durch systemische Gabe von rekombinantem, löslichen ACE2 bestätigt werden.

### Beispiel 3: Verlust der ACE2 Expression in Gegenwart inflammatorischer Zytokine

Die Nieren Zelllinie (Ceropithecus aethiops) Vero E6 exprimiert unter den üblichen Kulturbedingungen ACE2 als membranverankertes Glykoprotein. Vero E6 wurden für 48 Stunden mit 10 ng/ml IL-4, IFN-gamma oder TNF-alpha inkubiert und Änderungen hinsichtlich der ACE2 Oberflächenexpression mittels FACS Analytik unter Verwendung eines polyklonalen ACE2 spezifischen Ziegenantikörpers und eines ziegenspezifischen FITC markierten Antikörpers analysiert. In Fig. 6 sind die jeweiligen Histogramme dargestellt. Die zugehörige Auswertung ist in Tabelle 1 zusammengefaßt. Durch Inkubation mit IL-4, IFN-gamma oder TNF-alpha wurde die ACE2 Expression deutlich herabgesetzt. Während eine ACE2 Positivität von 51±3% in nicht stimulierten Zellen gemessen wurde, war diese auf 28±2, 22±1 und 39±2% jeweils nach IL-4, INF-gamma und TNF-alpha Stimulation herabgesetzt (Fig. 6).

**Tabelle 1: ACE2 spezifische FACS Analytik gemessen nach Inkubation von Vero E6 nach 48 stündiger Inkubation mit 10 ng/ml IL-4, IFN-gamma oder TNF-alpha im Vergleich zu einer unstimulierten Kontrollgruppe.**

| Stimulation | IL-4 | IFN-gamma | TNF-alpha | Ø |
|---|---|---|---|---|
| Positivität | 28±3 | 22±1 | 39±2 | 51±3 |
| Negativköntrolle | 5 | 2 | 4 | 6 |

### Beispiel 4: Attenuierung der Immunreaktivität von PBMCs

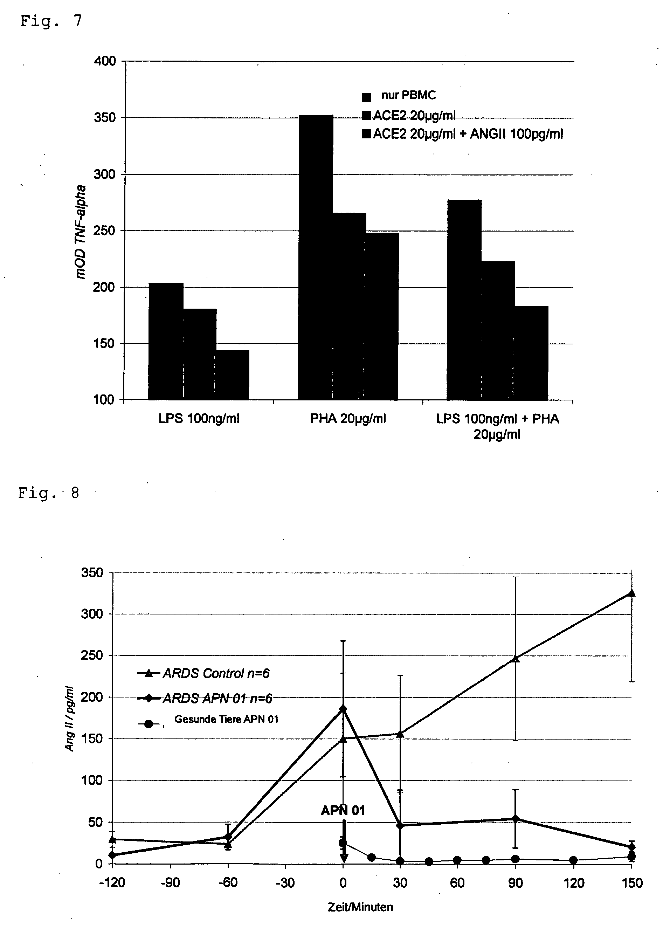

In diesem Beispiel wird der Effekt von ACE2 auf die Zytokinexpression stimulierter PBMCs (Periphere Mononukleare Blut Zellen) dargelegt. Es wurde eine PBMC Präparation und somit das gesamte Lymphozytenspektrum des Spenders im Ansatz verwendet, um das Zusammenspiel unterschiedlicher Lymphozyten zu ermöglichen. Einem gesunden Spender wurde Vollblut abgenommen und die darin enthaltenen PBMCs wurden durch Zentrifugation abgetrennt. Diese Zellen wurden in weiterer Folge mit stark immunogenen Substanzen, wie Lipo Poly Saccharid (LPS, 100 ng/ml) und Phyto Hämagglutinin (PHA, 20 µg/ml), und einer Kombination beider Substanzen in Gegenwart von Ang II, ACE2 und ACE2 mit Ang II stimuliert und bei 37°C für 16 Stunden inkubiert. Die Überstände wurden auf TNF-alpha untersucht und mit einem Kontrollansatz, der in Abwesenheit von ACE2 und Peptiden des RAS durchgeführt wurde, verglichen. Die Resultate dieses Versuches sind graphisch in Fig. 7 dargestellt: Die Inkubation mit LPS und PHA induzierte in allen Fällen die Sekretion von TNF-alpha. Die jeweiligen Kontrollansätze, die ohne ACE2 koinkubiert wurden, zeigten die höchsten TNF-alpha Konzentrationen (203, 352 und 278 mOD) jeweils nach LPS, PHA und der Kombinationsstimulation. In Gegenwart von ACE2 war das gemessene Signal in allen Gruppen deutlich geringer und erreichte nur noch mOD Werte von 181, 266, 223 in den jeweiligen Gruppen. Unter Anwesenheit von ACE2 und Ang II allerdings waren die gemessenen TNF-alpha Konzentrationen die geringsten und erreichten nur noch mOD 144, 247 und 183. Diese Ergebnisse zeigen auf, dass die Anwesenheit von ACE2 zu einer deutlich abgeschwächten Produktion inflammatorischer Zytokine führt, selbst wenn zur Stimulation besonders immunogene Substanzen wie LPS oder PHA verwendet werden. Dies bestätigt einen anti-inflammatorischen Effekt von ACE2. Erstaunlicherweise funktioniert der Mechanismus bereits ohne Anwesenheit von Ang II und wird in dessen Gegenwart verstärkt, was auf ein duales Prinzip hindeutet. Ein Teil des Effekts wird durch Ang II und dessen Abbauprodukt Ang 1-7 bewirkt, wobei ein weiterer Teil offensichtlich über den Abbau eines der anderen ACE2 Substrate funktioniert und nicht an vorhandenes Ang II gebunden ist (Fig. 7).

### Beispiel 5: Wiederherstellung der Ang II Titer des gesunden Organismus

In diesem Beispiel wurde aufgezeigt wie exogene ACE2 Gabe ein dereguliertes RAS wieder unter Kontrolle bringt. APN 01 (rekombinantes lösliches humanes ACE2) wurde hierfür in einem durch LPS Gabe induzierten Sepsis Modell verabreicht. Den Tieren wurde ab dem Zeitpunkt -120 Minuten kontinuierlich LPS infundiert, was zu einer massiven Inflammation und in weiterer Folge zu einer Sepsis führte. Es kam aufgrund der massiven Ausschüttung inflammtorischer Zytokine zum Abschalten der ACE2 Expression, was in weiterer Folge zu einer Akkumulation des inflammatorischen Peptides Ang II führte (siehe Fig. 8).

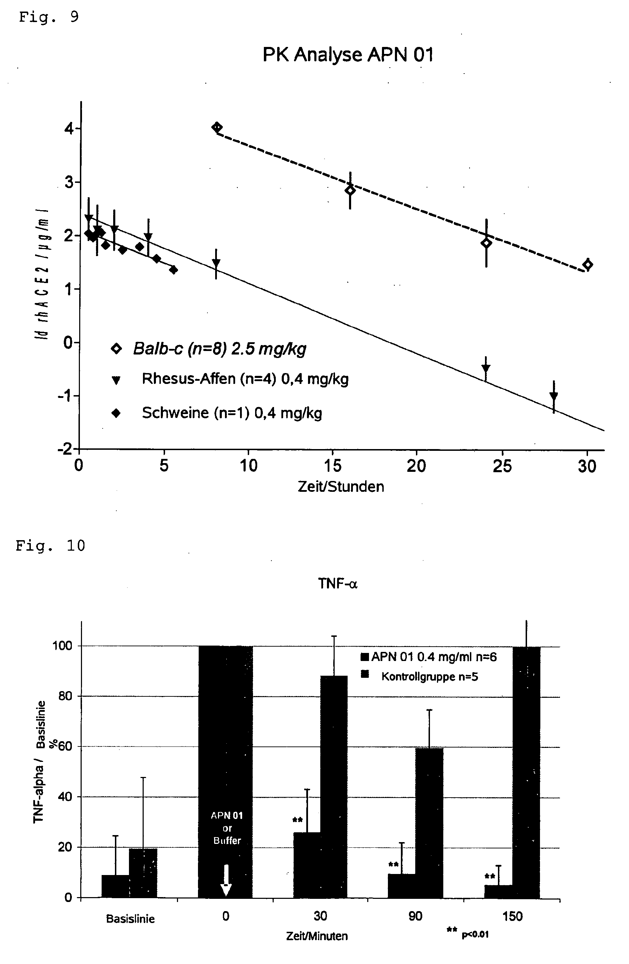

Ab dem Zeitpunkt 0 Minuten wurde APN 01 in einer Dosierung von 400 µg/kg als Bolus intra-venös verabreicht. Es kam sofort zu einer Abnahme von Ang II in der behandelten Gruppe., und die Ang II Titer pendelten sich binnen der darauf folgenden Stunde auf dasselbe Niveau, das auch in gesunden Tieren gemessen wurde, wieder ein. Ferner erbrachte die APN 01 Gabe derselben Dosis in gesunden Tieren ebenfalls einen kurzen Abfall der Ang II Titer, welche sich ebenfalls nach einer weiteren Stunde wieder den Werten der gesunden Tiere annäherten. Placebo behandelte Tiere zeigten hingegen einen weiter ansteigenden Ang II Wert bis zum Ende des Experimentes auf. Dieses erstaunliche Phänomen kann nur durch eine Wiederherstellung des hochregulierten RAS erklärt werden, da bis zum Ende des Experimentes nach wie vor aktives Enzym den Tieren systemisch zur Verfügung stand (siehe Fig. 9). Es wurde eine Halbwertszeit von ca. 8 Stunden gemessen.

### Beispiel 6: Attenuierung der Expression inflammatorischer Zytokine in der Sepsis

Im folgenden Beispiel wird gezeigt, wie die Konzentration des inflammatorischen Zytokins in einem Sepsismodell in Schweinen rapide zunimmt und nach ACE2 Gabe wieder auf das Niveau der gesunden Tiere zurückfällt. Den Tieren wurde ab dem Zeitpunkt -120 Minuten kontinuierlich LPS in hoher Dosierung infundiert, was zu einer massiven Inflammation und in weiterer Folge zu einer Sepsis führte. Es kam aufgrund der massiven Ausschüttung inflammtorischer Zytokine zum Abschalten der ACE2 Expression, was in weiterer Folge nicht nur zu einer Akkumulation des inflammatorischen Peptides Ang II, sondern ebenfalls des inflammatorischen Zytokins TNF-alpha führte (Fig. 10). Ab dem Zeitpunkt 0 Minuten wurde den Tieren (6 Tiere in der behandelten Gruppe, 5 Tiere in der Kontrollgruppe) entweder ACE2 in einer Dosierung von 0.4 mg/kg oder Pufferlösung als Bolus intravenös verabreicht. Während LPS weiters in derselben, hohen Dosierung kontinuierlich verabreicht wurde, wurden die Tiere für weitere 3 Stunden beobachtet und Serumproben gewonnen und auf TNF-alpha analysiert. Es konnte aufgezeigt werden, dass die TNF-alpha Konzentration in der Kontrollgruppe bis zum Ende des Experimentes erhöht blieb, während es in der ACE2 behandelten Gruppe bereits nach einmaliger ACE2 Verabreichung und bei fortwährender LPS Gabe zu einer deutlichen (p<0,001) Herabsetzung der TNF-alpha Konzentration kam. Es wurden, trotz massiver Sepsis, wieder in etwa dieselben Werte erreicht, die auch in gesunden Tieren gemessen wurden. Durch ACE2 Gabe konnte daher sogar in einem sehr aggressiven Sepsismodell die TNF-alpha Expression rapide auf das Niveau Gesunder herabgesetzt werden, und einer sich weiterhin potenzierenden Inflammation Einhalt geboten werden (Fig. 10).

### Beispiel 7: Attenuierung der Expression sämtlicher inflammatorischer Zytokine nach lokaler mechanischer Lungenschädigung

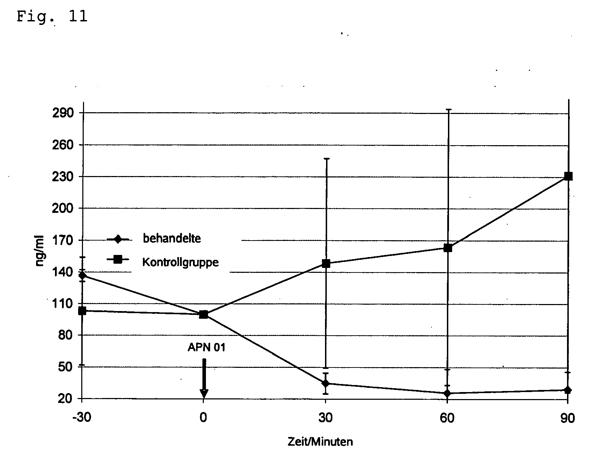

In diesem Beispiel wurde der Einfluss von systemisch verabreichtem ACE2 auf die Expression inflammatorischer Zytokine in einem Lungenschädigurigsmodell in Schweinen aufgezeigt. 14 Tiere wurden in dieser Placebo kontrollierten, verblindeten Studie berücksichtigt. Alle wurden in der ersten Phase des Experimentes einer dreimaligen Aspiration einer 20% Mekoniumlösung unterzogen, wobei aufgrund erhobener hämodynamischer Parameter eine vergleichbare Schädigung in allen Tieren induziert wurde. In der zweiten Phase des Experimentes, der therapeutischen, wurde der einen Hälfte der Tiere rekombinantes lösliches humanes ACE2 intravenös als Bolus in einer Dosierung von 0,4 mg/kg verabreicht. Die andere erhielt eine physiologische Kochsalzlösung. Es wurden zu den Zeitpunkten -30, 0, 30, 60, 90 und 150 Minuten Serumproben abgenommen, in welchen die Konzentrationen der bedeutendsten inflammatorischen Zytokine gemessen wurden. Der Zeitpunkt 0 war hierbei der Startpunkt der Therapie, zu welchem alle Tiere bereits ARDS Symptome zeigten. Wie in Fig. 11 verdeutlicht wird, gibt es einen sehr deutlichen Einfluss der ACE2 Verabreichung auf die Serumkonzentration von TNF-alpha. Während diese in der Placebogruppe von über 230 ng/ml stark ansteigt, fällt sie in der behandelten Gruppe binnen 30 Minuten nach Verabreichung auf unter 40 ng/ml und nähert sich um 90 Minuten nach Gabe 25 ng/ml an.

## Patentansprüche

1. ACE2-Protein oder eine ACE2 kodierende Nukleinsäure, zur therapeutischen Behandlung oder Vorbeugung einer Fibrose und/oder Lebererkrankung.

2. ACE2 oder Nukleinsäure nach Anspruch 1, **dadurch gekennzeichnet, dass** die Fibrose eine lokale Fibrose eines Gewebes oder Organs ist.

3. ACE2 oder Nukleinsäure nach Anspruch 1 oder 2, **dadurch gekennzeichnet, dass** die Fibrose Leberfibrosen, Lungenfibrosen, Bindegewebsfibrosen, Fibrose eines Muskels, Fibrose der Haut oder Nierenfibrose umfasst.

4. ACE2 oder Nukleinsäure nach einem der Ansprüche 1 bis 4 zur prophylaktischen Anwendung vor einer Fibrose und/oder Lebererkrankung.

5. ACE2 oder Nukleinsäure nach einem der Ansprüche 1 bis 4, **dadurch gekennzeichnet, dass** die Lebererkrankung zu Leberschäden oder Leberzellschäden führt.

6. ACE2 oder Nukleinsäure nach einem der Ansprüche 1 bis 5, **dadurch gekennzeichnet, dass** die Fibrose oder Lebererkrankung zusammen mit einer Inflammation in Verbindung steht.

7. ACE2 oder Nukleinsäure nach einem der Ansprüche 1 bis 6, **dadurch gekennzeichnet, dass** die Fibrose, Lebererkrankung oder Inflammation durch eine Infektion oder Wunde verursacht ist.

8. ACE2 oder Nukleinsäure nach einem der Ansprüche 1 bis 7, **dadurch gekennzeichnet, dass** das Protein rekombinantes ACE2 ist.

9. ACE2 oder Nukleinsäure nach einem der Ansprüche 1 bis 8, **dadurch gekennzeichnet, dass** das Protein wasserlösliches ACE2, insbesondere ohne Membrandomäne, ist.

10. ACE2 oder Nukleinsäure nach einem der Ansprüche 1 bis 9, **dadurch gekennzeichnet, dass** das Protein ACE2 von einem Säuger, vorzugsweise einem Menschen, einer Maus, einer Ratte, einem Hamster, einem Schwein, einem Primaten oder einem Rind ist.

11. Verwendung von ACE2 oder einer ACE2 kodierenden Nukleinsäure zur Herstellung einer pharmazeutischen Zusammensetzung zur Behandlung einer Fibrose und/oder Lebererkrankung.

12. Verwendung nach Anspruch 11, **dadurch gekennzeichnet, dass** die Fibrose und/oder Lebererkrankung bzw. ACE2 sowie dessen kodierende Nukleinsäure wie in einem der Ansprüche 1 bis 10 definiert sind.
